Europäisches Patentamt

European Patent Office

Office européen des brevets

Veröffentlichungsnummer: **0 487 776 A1**

# EUROPÄISCHE PATENTANMELDUNG

Anmeldenummer: **90122888.2**

Anmeldetag: **29.11.90**

Int. Cl.5: **A61N  1/08**

Veröffentlichungstag der Anmeldung:
**03.06.92 Patentblatt  92/23**

Benannte Vertragsstaaten:
**AT DE FR GB IT NL**

Anmelder: **SIEMENS AKTIENGESELLSCHAFT**
**Wittelsbacherplatz 2**
**W-8000 München 2(DE)**

Erfinder: **Kohl, Martin, Dipl-Ing.**
**Lerchenweg 1**
**W-8521 Bräuningshof(DE)**

Verfahren und Vorrichtung zum Bestimmen eines Parameters während der Abgabe eines elektrischen Impulses an biologisches Gewebe.

Nach dem Verfahren wird wenigstens ein variabler Parameter unabhängig von dem Betrag der Amplitude eines an biologisches Gewebe abgebbaren Impulses (19) eines RLC-Entladestromkreises (5) mit einer Vorrichtung bestimmt. Dazu wird in einer Meßeinrichtung (13), die an den RLC-Entladestromkreis angekoppelt ist und eine Auswerteschaltung (14) aufweist, der zeitliche Abstand zwischen zwei meßbaren Werten ($t_0$ bis $t_4$) des elektrischen Impulses (19) mit einer Zeitmeßschaltung (15) gemessen, dessen elektrisches Ausgangssignal der Auswerteschaltung (14) der Meßeinrichtung (13) zur Bestimmung eines Parameters des RLC-Entladestromkreises (5) zugeführt wird.

FIG 1

Die vorliegende Erfindung bezieht sich auf ein Verfahren zum Bestimmen wenigstens eines Parameters in einem RLC-Entladestromkreis während der Abgabe eines elektriscnen Impulses an biologisches Gewebe. Die Erfindung bezieht sich weiterhin auf eine Vorrichtung zur Durchführung des Verfahrens, wenigstens umfassend einen Speicherkondensator und eine Spule, welche über biologisches Gewebe zu einem RLC-Entladestromkreis verbindbar sind, an dem eine Meßeinrichtung mit einer Auswerteschaltung angekoppelt ist.

Ein derartiges Verfahren und eine derartige Vorrichtung sind aus der Zeitschrift Medical Electronics, Oktober 1986, Seiten 156 bis 158, bekannt und werden dort in Verbindung mit einem Defibrillator beschrieben. Vor allem aus medizinischen, aber auch aus rechtlicnen Gründen ist eine Messung nebst nachfolgender Dokumentation der bei Abgabe eines elektrischen Impulses zur Defibrillation im Entladestromkreis auftretenden wesentlichen Parameter erforderlich. Als variable Parameter treten im Entladestromkreis regelmäßig der Widerstand des biologischen Gewebes - z.B. der Patientenwiderstand - und die im Ladekondensator (= Speicherkondensator) gespeicherte Energie sowie die aus diesen beiden variablen Parametern resultierende und an den Patienten (lebendes biologisches Gewebe) abgegebene Energie auf. Bei der bekannten Vorrichtung ist zur Bestimmung derartiger Parameter eine Meßvorrichtung über einen Stromtransformator mit einem RLC-Entladestromkreis verbunden. Der Stromtransformator wird primärseitig von dem Entladestrom (abgegebener elektrischer Impuls) durchflossen. Die Sekundärseite des Transformators ist mit einem Lastwiderstand belastet und mit der Meßeinrichtung verbunden. Die Meßeinrichtung ist so ausgebildet, daß der Maximalwert der Sekundärspannung des Transformators gemessen und in einem Mikroprozessor zur Ermittlung des Patientenwiderstandes ausgewertet wird.

Folglich hängt die Genauigkeit dieser Meßmethode von dem Betrag der Amplitude der Sekundärspannung des induzierten Stromes ab. Der Betrag der Amplitude ist - abgesehen vom Übersetzungsverhältnis des Transformators, der Kapazität des Ladekondensators und der Induktivität der Spule sowie deren Widerstand (feste Parameter) - von zwei variablen Parametern abhängig. Ein variabler Parameter ist der zu ermittelnde Patientenwiderstand und der andere variable Parameter ist die jeweils im Ladekondensator gespeicherte und regelmäßig einstellbare Energie. Für die Bestimmung des Patientenwiderstandes ist es sonach notwendig, zugleich die im Ladekondensator gespeicherte Energie zu bestimmen und bei der Auswertung den gemessenen Betrag der Amplitude der Sekundärspannung zu berücksichtigen. Das ist aufwendig und führt vor allem zu größeren Meßtoleranzen.

Der Erfindung liegt die Aufgabe zugrunde, ein Verfahren und eine Vorrichtung der eingangs genannten Art anzugeben, womit wenigstens ein variabler Parameter im RLC-Entladestromkreis unabhängig von dem Betrag der Amplitude des abgegebenen elektrischen Impulses bestimmbar ist.

Diese Aufgabe wird nach einem Verfahren gemäß Anspruch 1 und einer Vorrichtung nach Anspruch 7 gelöst.

Ein wesentlicher Vorteil der Erfindung besteht darin, daß ein variabler Parameter in dem RLC-Entladestromkreis, z.B. der Patientenwiderstand, völlig unabhängig von dem Betrag der Amplitude des abgegebenen elektrischen Impulses bestimmbar ist. Das wird erreicht, indem der zeitliche Abstand zwischen zwei meßbaren ( = charakteristischen) Werten des abgegebenen elektrischen Impulses gemessen und ausgewertet wird. Ein charakteristischer Wert des Impulses kann z.B. der Zeitpunkt sein, in welchem der Maximalwert der Amplitude erreicht ist. Die Erfindung geht folglich von der Erkenntnis aus, daß der Zeitpunkt, in welchem ein bestimmter meßbarer Wert der Amplitude des abgegebenen Impulses auftritt, völlig unabhängig von dem Betrag der Amplitude erreicht wird. Während dagegen der Betrag der Amplitude des abgegebenen Impulses sowohl von der in dem Ladekondensator gespeicherten Energie als auch von dem Patientenwiderstand abhängig ist, ist die erfindungsgemäß zur Bestimmung eines variablen Parameters ausgewählte Zeitdauer von der im Ladekondensator gespeicherten Energie völlig unabhängig. Da die festen Parameter im RLC-Entladestromkreis, nämlich die Kapazität des Speicherkondensators, die Induktivität der Spule und deren ohmscher Innenwiderstand bekannt und nicht variabel ausgebildet sind, kann z.B. nach einer Variante der Erfindung aus diesen festen, bekannten Parametern und aus der gemessenen variablen Zeitdauer vor allem der Widerstand des biologischen Gewebes (Patientenwiderstand) mit hoher Genauigkeit in einer Meßeinrichtung bestimmt werden. In der Meßeinrichtung kann nach einer anderen Variante der Erfindung z.B. auch eine Wertetabelle gespeichert sein, in welcher einer jeweils meßbaren Zeitdauer ein bestimmter Widerstandswert des biologischen Gewebes zugeordnet ist.

Weitere Vorteile und Einzelheiten der Erfindung ergeben sich aus der nachfolgenden Beschreibung von Ausführungsbeispielen anhand der Zeichnungen und in Verbindung mit den Ansprüchen.

Es zeigen:

Figur 1 ein prinzipielles Blockschaltbild einer erfindungsgemäßen Vorrichtung,

Figur 2 eine aperiodisch gedämpfte Schwingung, die als elektrischer Impuls an biologisches Gewebe abgebbar ist,

Figur 3 ein aus dem elektrischen Impuls gemäß Figur 2 abgeleitetes differenziertes Signal,

Figur 4 ein Teilschaltbild zu der erfindungsgemäßen Vorrichtung gemäß Figur 1 mit detaillierterer Darstellung einer Zeitmeßschaltung und

Figur 5 ein Rechtecksignal, das innerhalb der Zeitmeßschaltung gemäß Figur 4 erzeugt wird.

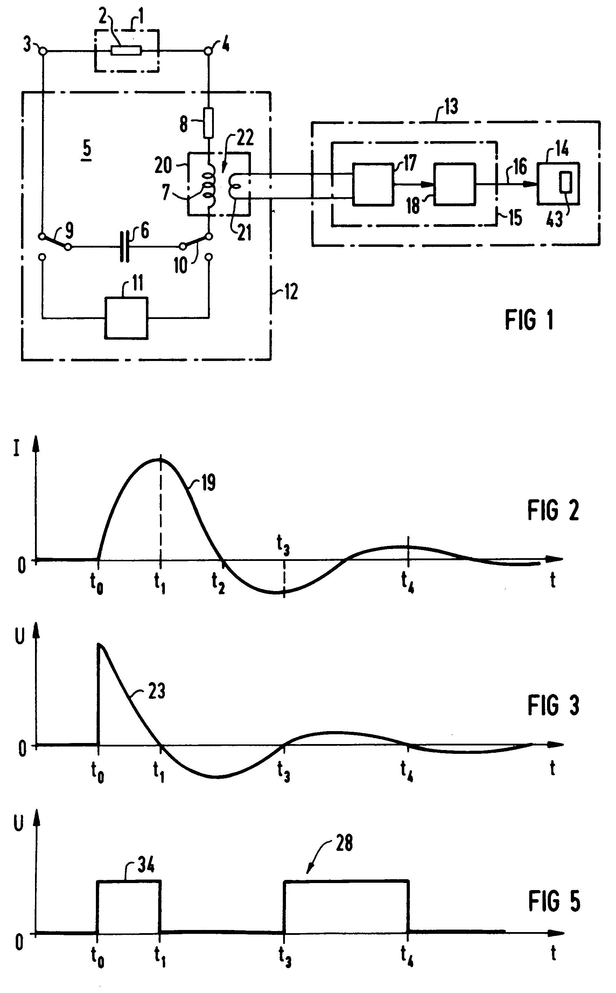

Figur 1 zeigt eine Vorrichtung, mit der wenigstens ein Parameter während der Abgabe eines elektrischen Impulses an biologisches Gewebe 1 bestimmbar ist. Das biologische Gewebe 1, z.B. von einem Patienten, weist einen ohmschen Widerstand 2 auf, der in Abhängigkeit von dem Gewebe 1 des Patienten variieren kann. Das biologische Gewebe 1 ist über Elektroden 3 und 4 mit einem RLC-Entladestromkreis 5 verbunden, der einen Speicherkondensator 6 und eine Spule 7 aufweist. Des weiteren ist noch ein Widerstand 8 im RLC-Entladestromkreis 5 dargestellt, der den Innenwiderstand der Spule 7 symbolisiert. Der Speicherkondensator 6 ist zum Aufladen über Schaltmittel 9 und 10, die auch elektronisch ausgebildet und gesteuert sein können, mit einer Auflade- und Steuerschaltung 11 verbindbar. Die Auflade- und Steuerschaltung 11 dient zum Laden des Speicherkondensators 6 und zum Steuern der Schaltmittel 9 und 10, wodurch auch die Abgabe eines elektrischen Impulses aus dem RLC-Entladestromkreis 5 an das biologische Gewebe 1 steuerbar ist. Der RLC-Entladestromkreis 5 und die Auflade- und Steuerschaltung 11 können als ein Bestandteil 12 der erfindungsgemäßen Vorrichtung in einem Reizstromgerät, insbesondere in einem Defibrillator, verwendet sein, wobei der Defibrillator auch in den Patienten implantierbar und über die Elektroden (Anschlüsse) 3 und 4 mit dem biologischen Gewebe des Herzmuskels verbindbar sein kann.

An den RLC-Entladestromkreis 5 ist eine Meßeinrichtung 13 angekoppelt, die eine Auswerteschaltung 14 aufweist. Die Meßeinrichtung 13 weist des weiteren eine Zeitmeßschaltung 15 auf. Die Zeitmeßschaltung 15 ist so ausgebildet, daß der zeitliche Abstand zwischen zwei charakteristischen Werten des an das biologische Gewebe 1 abgebbaren elektrischen Impulses meßbar und als ein elektrisches Signal über die Leitung 16 an die Auswerteschaltung 14 der Meßeinrichtung 13 abgebbar ist. In der Auswerteschaltung 14 wird aus dem über die Leitung 16 empfangenen Zeitsignal ein Parameter in dem RLC-Entladestromkreis 5, insbesondere der Wert des Widerstandes 2 des biologischen Gewebes 1, z.B. mit Hilfe eines Mikroprozessors 43, bestimmt.

Die Zeitmeßschaltung 15 kann mehrere Baugruppen enthalten. Eine erste Baugruppe 17 dient zur Erkennung von charakteristischen (= meßbaren) Werten des an das biologische Gewebe 1 abgebbaren elektrischen Impulses. In Abhängigkeit von solchen erkannten charakteristischen Werten kann die Baugruppe 17 dann eine Zählschaltung 18 steuern, die z.B. Taktimpulse zählt. So kann die Zählschaltung 18 bei Vorliegen eines ersten charakteristischen Wertes mit einem Signal aus der Baugruppe 17 gestartet und bei Vorliegen eines zweiten charakteristischen Wertes gestoppt werden. Die Anzahl der gezählten Impulse stellt ein Maß für den zeitlichen Abstand (Zeitdauer) zwischen beiden charakteristischen Werten dar und wird als elektrisches Signal über die Leitung 16 der Auswerteschaltung 14 zugeführt. Eine derartige Zeitmeßschaltung 15 kann mit geringem Aufwand bei hoher Meßgenauigkeit (geringe Meßtoleranz) hergestellt werden.

In Figur 2 ist ein an das biologische Gewebe 1 abgebbarer typischer elektrischer Impuls 19 als Stromverlaufskurve dargestellt. Der elektrische Impuls 19 hat die Charakteristik einer gedämpften Schwingung. Andere Impulsformen, z.B. eine aperiodisch gedämpfte Schwingung, können in Abhängigkeit von den elektrischen Parametern im RLC-Entladestromkreis 5 der Figur 1 auch vorkommen. Der elektrische Impuls 19 weist als einfach feststellbare charakteristische Werte z.B. den Impulsanfang zum Zeitpunkt $t_0$, einen Maximalwert der Amplitude zum Zeitpunkt $t_1$ und einen Nulldurchgang zum Zeitpunkt $t_2$ sowie kleine Maximalwerte zu den Zeitpunkten $t_3$ und $t_4$ auf. Folglich kann die erste Baugruppe 17 in Figur 1 so ausgebildet sein, daß sie z.B. den Impulsanfang $t_0$ und/oder den Maximalwert der Amplitude zum Zeitpunkt $t_1$ und/oder den Nulldurchgang der Amplitude zum Zeitpunkt $t_2$ erkennt. Sämtliche Zeitpunkte $t_1$ bis $t_4$ sind abhängig von der Kapazität des Speicherkondensators 6, von der Induktivität der Spule 7, von dem Innenwiderstand 8 der Spule 7 (feste Parameter) und von dem wechselnden Wert des Widerstandes 2 des biologischen Gewebes 1 (variabler Parameter).

Um die Vorrichtung gemäß Figur 1 zu kalibrieren, können vom Hersteller zwischen den Elektroden 3 und 4 zunächst hochgenaue Meßwiderstände angeordnet werden und die bei Abgabe eines Impulses 19 gemessenen Zeiten zwischen zwei charakteristischen Werten, z.B. zwischen $t_0$ und $t_1$, in der Auswerteschaltung 14 nach einer Variante der Erfindung als Wertetabelle in Relation zum Wert des Meßwiderstandes in dem Mikroprozessor 43, abgelegt werden. Zwischenwerte können durch Interpolation errechnet werden. Es ist nach einer anderen Variante der Erfindung aber auch möglich, die jeweilige Zeitdauer bis zum Erreichen eines charakteristischen Wertes des Impulses 19 in Abhängigkeit von dem wechselnden Wert des Widerstandes 2 und von den festen Werten der Spule 7 nebst deren Widerstand 8 und des Kondensators 6 im Mikroprozessor 43 zu errechnen. Die mathematischen Grundlagen zum Verhalten des RLC-Entladestromkreises sind z.B. aus der Zeitschrift "International Medical Device & Diagnostic Industry" Mai/Juni 1990, Seite 48, bekannt.

Die in Figur 1 dargestellte Meßeinrichtung 13 kann in Ausbildung der Erfindung über ein den elektrischen Impuls 19 differenzierendes Mittel 20 an den RLC-Entladestromkreis 5 angekoppelt werden. Dazu kann z.B. eine kapazitive Ankopplung über einen Kondensator (nicht dargestellt) vorgesehen sein. Besonders vorteilhaft ist es, wenn die Zeitmeßschaltung 15 eingangsseitig mit der Sekundärwicklung 21 eines Transformators 22 verbunden ist, der als ein den elektrischen Impuls 19 differenzierendes Mittel 20 ausgebildet ist.

Um die Anzahl und Größe der die Meßgenauigkeit beeinflussenden Bauteile gering zu halten, ist die Spule 7 im RLC-Entladestromkreis 5 als Primärwicklung des Transformators 22 ausgebildet. Zur weiteren Verbesserung der Meßgenauigkeit ist der Transformator 22 als ein im Leerlauf betreibbarer Transformator hochohmig mit der Zeitmeßschaltung 15 verbunden. Dadurch ist es auch möglich, die Sekundärwicklung 21 mit nur wenigen Windungen, z.B. zwei Windungen, auszubilden, wodurch bereits eine ausreichend genau meßbare Leerlaufspannung an der Sekundärwicklung entsteht, auch dann, wenn der Transformator 22 als Lufttransformator - wie dargestellt - ausgebildet ist.

Die Leerlaufspannung der Sekundärwicklung 21 des Lufttransformators 22 ist in Figur 3 dargestellt. Diese Leerlaufspannung bildet ein aus dem elektrischen Impuls 19 abgeleitetes und differenziertes Signal 23. Dieses differenzierte Signal 23 entspricht mathematisch betrachtet folglich der ersten Ableitung der Kurve des elektrischen Impulses 19. In den Figuren 2 und 3 ist das durch eine zeitliche Übereinstimmung des ersten Wendepunktes der Kurve 19 im Zeitpunkt $t_1$ mit einem ersten Nulldurchgang der Kurve 23 dargestellt. Folglich entspricht der untere Wendepunkt der Kurve 19 zum Zeitpunkt $t_3$ einem Nulldurchgang der Kurve 23 zum gleichen Zeitpunkt. Die Ableitung eines differenzierten Signals aus dem elektrischen Impuls 19 hat den wesentlichen Vorteil, daß der Zeitpunkt $t_1$ - der dem Zeitpunkt entspricht, in welchem der erste Maximalwert der Amplitude des Impulses 19 erreicht ist - mit höchster Genauigkeit durch den zeitlich entsprechenden Nulldurchgang in Kurve 23 bestimmbar ist. Des weiteren ist von Vorteil, daß der durch den ersten Maximalwert gegebene Zeitpunkt $t_1$ auch bei aperiodisch gedämpften Formen des Impulses 19 eindeutig erfaßbar ist.

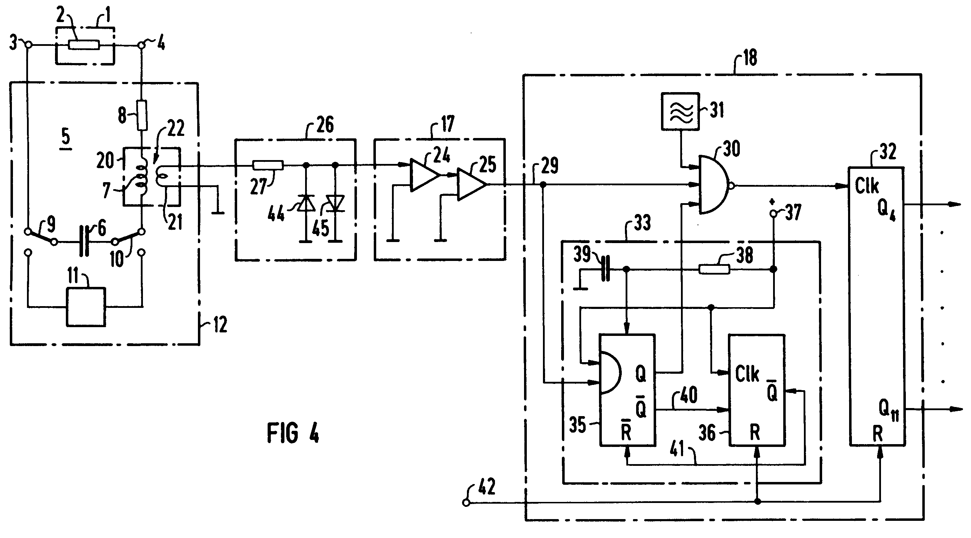

In Figur 4 sind die Zählschaltung 18 und die erste Baugruppe 17 der zu Figur 1 beschriebenen Zeitmeßschaltung 15 als eine spezielle Ausführung dargestellt. Die Baugruppe 17 enthält zwei Komparatorschaltungen 24 und 25, die über eine Begrenzerschaltung 26 mit dem zu Figur 1 beschriebenen RLC-Entladestromkreis 5 in dem Bestandteil 12 der erfindungsgemäßen Vorrichtung verbunden sind. Die Begrenzerschaltung 26 enthält einen Begrenzungswiderstand 27 und zwei antiparallel geschaltete Dioden 44 und 45. Mit der nicht zwingend erforderlichen Begrenzerschaltung 26 wird die in Figur 3 auf den Zeitpunkt $t_0$ folgende Spannungsspitze des differenzierten Signales 23 begrenzt, um den Eingang der nachfolgenden Komparatorschaltung 24 vor Überspannungen zu schützen. Der Ausgang des Komparators 24 ist mit einem Eingang des Komparators 25 verbunden, wodurch jeder Nulldurchgang des differenzierten Signals gemäß Kurve 23 in Figur 3 besonders genau bestimmt werden kann.

Durch die spezielle Ausbildung der Baugruppe 17 gemäß Figur 4 entsteht an deren Ausgang ein Rechtecksignal 28, das in Figur 5 dargestellt ist. Mit diesem Rechtecksignal 28 wird über eine Leitung 29 eine Torschaltung 30 in der Zählschaltung 18 angesteuert (Figur 4). Über einen anderen Eingang erhält die Torschaltung 30 aus einem Taktgenerator 31 Taktimpulse. Die Taktimpulse werden in einem Zählerbauteil 32 während der Zeitdauer gezählt, während der die Torschaltung 30 z.B. durch das Rechtecksignal 28 (Impuls 34) auf der Leitung 29 geöffnet worden ist. Als Zählerbauteil eignet sich z.B. ein 11-Bit-Zähler-IC mit der Typenbezeichnung 4020, wobei dieser integrierte Schaltkreis 4020 Parallelausgänge Q4 bis Q11 aufweist. Das elektrische Ausgangssignal aus dem Zählerbauteil 32 kann dann über eine entsprechend vielpolig ausgebildete Leitung 16 der Auswerteschaltung 14 zur Bestimmung eines Parameters zugeführt werden, wie es zu der Figur 1 bereits erwähnt ist.

Wenn der an das biologische Gewebe 1 abgegebene elektrische Impuls die Form einer gedämpften Schwingung gemäß Kurve 19 in Figur 2 haben kann, ist es zweckmäßig, in weiterer Ausgestaltung der Erfindung eine Zusatzschaltung 33 gemäß Figur 4 vorzusehen. Mit dieser Zusatzschaltung 33 wird die Torschaltung 30 zusätzlich derart gesteuert, daß nur während der Zeitdauer eines ersten Impulses 34 ($t_0$ bis $t_1$) des Rechtecksignales 28 gemäß Figur 5 Zählimpulse aus dem Taktgenerator 31 gemäß Figur 4 zu dem Zählerbauteil 32 gelangen können. Dazu weist die Zusatzschaltung 33 zwei Kippstufen auf, wobei eine Kippstufe als monostabiler Multivibrator 35 und eine andere Kippstufe als bistabiler Multivibrator 36 ausgebildet ist. Die monostabile Kippstufe 35 erhält über den Anschluß 37 eine positive Betriebsspannung, die auch ein RC-Zeitglied, gebildet aus einem Widerstand 38 und einem Kondensator 39, speist. Die monostabile Kippstufe 35 wird durch die erste Flanke im Zeitpunkt $t_0$ des Impulses 34 des Rechtecksignales 28 gestartet, das auf der Leitung 29 bei Abgabe eines Impulses 19 an das biologische Gewebe 1 entsteht. Dadurch wird an einem Ausgang Q der Kippstufe 35 ein Ausgangssignal erzeugt, mit dem die Torschaltung 30 während der durch das RC-Glied 38, 39 einstellbaren Standzeit der Kippstufe 35 geöffnet ist. Ein invertierender Ausgang $\overline{Q}$ ist über eine Leitung 40 mit einem Steuereingang CLK der bistabilen Kippstufe 36 verbunden. Die bistabile Kippstufe 36 sperrt sodann über eine Leitung 41 die monostabile Kippstufe 35, so daß diese von eventuell nachfolgenden Impulsen des Rechtecksignales 28 nicht mehr auslösbar ist. Vor der Abgabe eines weiteren elektrischen Impulses 19 an das biologische Gewebe 1 kann die Zusatzschaltung 33 und der Zähler 32 durch ein über den Anschluß 42 eingebbares Resetsignal, z.B. aus der Auflade- und Steuerschaltung 11, wieder in den Ausgangszustand zurückgesetzt werden. Als monostabile Kippstufe 35 kann z.B. ein Baustein mit der Typenbezeichnung 4538 und als bistabile Kippstufe ein Baustein mit der Typenbezeichnung 4013 verwendet werden.

In jedem an das biologische Gewebe 1 abgebbaren elektrischen Impuls 19 stellen der Impulsanfang zum Zeitpunkt $t_0$ und der Zeitpunkt $t_1$ im Maximum der Impulsamplitude besonders charakteristische und genau meßbare Werte eines derartigen Impulses dar. Die Zeitdauer, die zwischen dem Zeitpunkt $t_0$ und dem Zeitpunkt $t_1$ liegt, ist folglich gemäß der vorliegenden Erfindung mit großer Genauigkeit meßbar und nicht von der im Speicherkondensator 6 gespeicherten Energie abhängig. Ein weiterer Parameter in dem RLC-Entladestromkreis 5, z.B. die an das biologische Gewebe 1 abgegebene Energie ( = W2), kann ebenfalls mit hoher Genauigkeit ermittelt werden. Dazu wird aus dem genau gemessenen Wert des Widerstandes 2 ( = R2) des biologischen Gewebes 1 sowie aus dem Spulenwiderstand 8 ( = R8) und aus der im Speicherkondensator 6 gespeicherten Energie ( = W1) die an das biologische Gewebe 1 abgegebene Energie ( = W2) bestimmt nach der Formel: W2 = W1 x R2 / (R2 + R8). Diese Bestimmung eines weiteren Parameters ist ohne besonderen Aufwand z.B. mit dem der Auswerteschaltung 14 zugeordneten Mikroprozessor 43 möglich. Da der Wert des Widerstandes 2 ( = R2) erfindungsgemäß unabhängig von der im Speicherkondensator 6 gespeicherten Energie W1 bestimmt wurde, ist schon deshalb auch die an das biologische Gewebe 1 abgegebene Energie W2 mit größerer Genauigkeit bestimmbar.

**Patentansprüche**

1. Verfahren zum Bestimmen von wenigstens einem Parameter in einem RLC-Entladestromkreis (5) während der Abgabe eines elektrischen Impulses (19) an biologiches Gewebe (1) mit folgenden Verfahrensschritten:
   - mit einer Meßeinrichtung (13) wird der zeitliche Abstand zwischen zwei meßbaren Werten ($t_0$ bis $t_4$) des elektrischen Impulses (19) gemessen,
   - der gemessene zeitliche Abstand wird in der Meßeinrichtung (13) zur Bestimmung eines Parameters des RLC-Entladestromkreises (5) ausgewertet.

2. Verfahren nach Anspruch 1, wobei der zeitliche Abstand zwischen zwei meßbaren Werten ($t_0$ bis $t_4$) des elektrischen Impulses (19) aus einem daraus differenzierten Signal (23) bestimmt wird.

3. Verfahren nach Anspruch 1 oder 2, wobei als ein erster meßbarer Wert ($t_0$ bis $t_3$) des elektrischen Impulses der Anfang ($t_0$) dieses Impulses (19) oder der Anfang ($t_0$) aus dem daraus differenzierten Signal (23) gewählt wird.

4. Verfahren nach einem der Ansprüche 1 bis 3, wobei als ein zweiter meßbarer Wert ($t_1$ bis $t_4$) des elektrischen Impulses (19) ein Nulldurchgang ($t_2$) dieses Impulses oder ein Nulldurchgang ($t_1$, $t_3$, $t_4$) aus einem daraus differenzierten Signal (23) gewählt wird.

5. Verfahren nach einem der Ansprüche 2 bis 4, wobei als ein meßbarer Wert ($t_0$ bis $t_4$) des elektrischen Impulses (19) der erste Nulldurchgang ($t_1$) in dem differenzierten Signal (23) gemessen wird, der zeitlich einem ersten Maximalwert der Amplitude des elektrischen Impulses (19) entspricht.

6. Verfahren nach Anspruch 1, wobei aus den festen, bekannten Parametern (Kapazität des Speicherkondensators 6, Induktivität der Spule 7, ohmscher Innenwiderstand 8) des RLC-Entladestromkreises und dem gemessenen zeitlichen Abstand ein Parameter (Widerstand 2) des biologischen Gewebes (1) bestimmt wird.

7. Vorrichtung zur Durchführung des Verfahrens nach einem der Ansprüche 1 bis 6, wenigstens umfassend einen Speicherkondensator (6) und eine Spule (7), welche über biologisches Gewebe (1) zu einem RLC-Entladestromkreis (5) verbindbar sind, an dem eine Meßeinrichtung (13) mit einer Auswerteschaltung (14) angekoppelt ist, wobei die Meßeinrichtung eine Zeitmeßschaltung (15) aufweist, mit welcher der zeitliche Abstand zwischen zwei charakteristischen Werten ($t_0$ bis $t_4$) des elektrischen Im-

pulses (19) meßbar und als ein elektrisches Signal an die Auswerteschaltung (14) der Meßeinrichtung (13) abgebbar ist.

8. Vorrichtung nach Anspruch 7, wobei die Meßeinrichtung (13) über ein den elektrischen Impuls (19) differenzierendes Mittel (20) an den RLC-Entladestromkreis (5) angekoppelt ist.

9. Vorrichtung nach Anspruch 7 oder 8, wobei die Zeitmeßschaltung (15) an den RLC-Entladestromkreis (5) über einen Transformator (22) angekoppelt ist, der als das den elektrischen Impuls (19) differenzierende Mittel (20) ausgebildet ist.

10. Vorrichtung nach Anspruch 9, wobei die Spule (7) im RLC-Entladestromkreis (5) als Primärwicklung (20) des Transformators (22) ausgebildet ist.

11. Vorrichtung nach Anspruch 9 oder 10, wobei mit der Zeitmeßschaltung (15) ein Nulldurchgang ($t_1$, $t_3$, $t_4$) an der Sekundärwicklung (21) des Transformators (22) meßbar ist, aus dessen zeitlichem Abstand zum Anfang ($t_0$) des elektrischen Impulses (19) ein Parameter im RLC-Entladestromkreis (5) bestimmbar ist.

12. Vorrichtung nach Anspruch 11, wobei die Zeitmeßschaltung (15) eine Komparatorschaltung (24, 25) zur Bestimmung eines Nulldurchganges aufweist, die eingangsseitig mit der Sekundärwicklung (21) des Transformators (22) verbunden ist.

13. Vorrichtung nach Anspruch 11 oder 12, wobei die Zeitmeßschaltung (15) eine Zählschaltung (18) aufweist, die derart steuerbar ist, daß durch den ersten Nulldurchgang ($t_1$) des differenzierten elektrischen Signales (23) der zeitliche Abstand zwischen dem Anfang ($t_0$) und dem Maximalwert der Amplitude des elektrischen Impulses (19) bestimmbar ist.

14. Vorrichtung nach einem der Ansprüche 9 bis 13, wobei der Transformator (22) im wesentlichen als ein im Leerlauf betreibbarer Transformator ausgebildet und hochohmig mit der Zeitmeßschaltung (15) verbunden ist.

15. Vorrichtung nach einem der Ansprüche 11 bis 14, wobei jeder meßbare Nulldurchgang ($t_1$, $t_3$, $t_4$) an der Sekundärwicklung (21) aus deren Leerlaufspannung bestimmbar ist.

16. Vorrichtung nach einem der Ansprüche 11 bis 15, wobei der Transformator (22) als Lufttransformator ausgebildet ist.

17. Vorrichtung nach einem der Ansprüche 11 bis 16, wobei die Sekundärwicklung (21) des Transformators (22) eine Windungszahl aufweist, die klein gegenüber der Windungszahl der Primärwicklung (7) gewählt ist.

18. Vorrichtung nach einem der Ansprüche 8 bis 17, deren Auswerteschaltung (14) einen Mikroprozessor (43) aufweist, in welchem eine Wertetabelle abgelegt ist, in welcher meßbaren Zeitpunkten ($t_1$, $t_3$, $t_4$) von Nulldurchgängen des differenzierten elektrischen Signales (23) Widerstandswerte des biologischen Gewebes (1) zugeordnet sind.

19. Vorrichtung nach Anspruch 18, bei der im Mikroprozessor (43) der jeweils bestimmte Wert des Widerstandes (2) des biologischen Gewebes (1) durch die Summe aus diesem Widerstandswert des Patientengewebes (1) und dem bekannten Spulenwiderstand (8) im Entladestromkreis (5) dividierbar ist, wobei durch Multiplikation des erhaltenen Ergebnisses mit der im Ladekondensator (6) gespeicherten Energie die an das biologische Gewebe (1) abgegebene Energie bestimmbar ist.

FIG 1

FIG 2

FIG 3

FIG 5

FIG 4

EP 0 487 776 A1

**Europäisches Patentamt**

# EUROPÄISCHER RECHERCHENBERICHT

Nummer der Anmeldung

EP    90 12 2888

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | IEEE TRANSACTIONS ON INSTRUMENTATION AND MEASUREMENT. vol. im-31, no. 1, März 1982, NEW YORK US Seiten 28 - 31; V. K. SHARMA et al.: "A reliable microprocessor-based defibrillator analyzer" * das ganze Dokument * | 1, 3-7, 18, 19 | A61N1/08 |
| A | | 9, 10 | |
| | --- | | |
| Y | MEDICAL AND BIOLOGICAL ENGINEERING AND COMPUTING. vol. 16, no. 2, März 1978, STEVENAGE GB Seiten 169 - 178; J. W MACHIN: "Thoracic impedance of human subjects" * das ganze Dokument * | 1, 3-7, 18, 19 | |
| A | | 2, 8 | |
| | --- | | |
| A | GB-A-2085593 (HEWLETT-PACKARD) * das ganze Dokument * | 1, 7 | |
| | --- | | |
| A | EP-A-0315368 (HEWLETT-PACKARD) * das ganze Dokument * | 1, 7 | **RECHERCHIERTE SACHGEBIETE (Int. Cl.5 )** |
| | ----- | | A61N |

Der vorliegende Recherchenbericht wurde für alle Patentansprüche erstellt

| Recherchenort | Abschlußdatum der Recherche | Prüfer |
|---|---|---|
| DEN HAAG | 01 AUGUST 1991 | FERRIGNO A. |